# EUROPEAN PATENT APPLICATION

(11) **EP 4 339 964 A1**
(43) Date of publication of application: **20.03.2024**
(21) Application number: 22195365.6
(22) Date of filing: 13.09.2022
(51) Int. Cl.: G16H 40/20, G16H 40/67, G16H 50/70, G16H 70/20, G16H 30/20, G06N 3/04, G06Q 10/20

(54) **A MONITORING AGENT FOR MEDICAL DEVICES**

(71) Applicant: Koninklijke Philips N.V., 5656 AG Eindhoven (NL)
(72) Inventor: SOUBHIK, Paul, Eindhoven (NL); NAIK, Sarif Kumar, Eindhoven (NL)
(74) Representative: Philips Intellectual Property & Standards

(57) **Abstract**

Proposed concepts aim to provide schemes, solutions, concepts, designs, methods and systems pertaining to monitoring medical devices. In particular, usage data of the medical device and other (related) medical devices is gathered. Tis data is used to learn normal operation values of the medical device. Accordingly, any deviations of the operation of the medical device from the normal operation values and a standard operation policy may be determined. The deviation is then used to generate an operation issue signal (i.e. a user alert, a correction action, etc.). In this way, operation issues may be automatically detected and potentially corrected at an early stage.

## Description

### FIELD OF THE INVENTION

The present invention relates to the field of monitoring agents, and more particularly to the field of agents for monitoring medical devices.

### BACKGROUND OF THE INVENTION

Medical devices typically require skilled technicians for maintenance and service. However, due to a limited workforce, owners of such devices have to wait a long period of time for diagnosis and rectification of reported problems. Moreover, medical devices are often essential for the swift diagnosis and treatment of patients. As such, the expedient fixing of medical devices is key for providing effective care.

In order to know what the characteristics of wrong/faulty behavior of the medical device (and components thereof) is, it is important to know what a typical/normal usage pattern for the device is. Skilled technicians are trained to be aware of a normal usage pattern of medical devices, but technicians are not present for every deviation from normal behavior.

Accordingly, automatic detection of potential future problems would resolve many issues. Furthermore, fixing issues in situ may save the time of technicians, meaning that they may be able to assign time more effectively to more complex issues. In addition, early diagnosis and rectification of faulty behavior may prevent more severe breakdowns, also saving time and cost.

Moreover, in cases where the fault is due to a specialized component, a replacement component may take a long time to become available. Thus, any time that can be saved may prevent the device from being offline for an unmanageable amount of time.

Therefore, there exists a present need for a means for effectively monitoring medical devices in order to detect, and potentially correct, faulty behavior.

### SUMMARY OF THE INVENTION

The invention is defined by the claims.

According to examples in accordance with an aspect of the invention, there is provided an agent for monitoring a medical device, the agent comprising:
an interface configured to obtain usage data describing an operation of a medical device;
a network module configured to obtain usage data describing an operation of at least one other medical device related to the medical device;
a learning module configured to determine normal operation values of the medical device based on the usage data of the medical device, and the usage data of the at least one other medical device, and
an analysis module configured to:
   compare the usage data of the medical device with the normal operation values of the medical device and a standard operation policy; and
   generate, based on the comparison, an operation issue signal describing a detected discrepancy between the usage data of the medical device with the normal operation values of the medical device.

There are thus proposed concepts pertaining to monitoring medical devices. In particular, usage data of the medical device and other (related) medical devices is gathered. Tis data is used to learn normal operation values of the medical device. Accordingly, any deviations of the operation of the medical device from the normal operation values and a standard operation policy may be determined. The deviation is then used to generate an operation issue signal (i.e. a user alert, a correction action, etc.). In this way, operation issues may be automatically detected and potentially corrected at an early stage.

Indeed, by leveraging both data from the medical device being monitored by the agent and data from other (related) medical devices, an accurate picture of a normal/typical operation of the medical device. Determining accurate normal operation values is vital to the accurate detection of erroneous operation of a medical device (i.e. due to a faulty component, incorrect operation conditions/parameters, etc.). Indeed, for the detected discrepancy/deviation of the usage of the medical device to accurately reflect an error state, the normal operation values must accurately define how the medical device should run.

Furthermore, a same type of medical device may be used in a variety of environments, in a variety of operation modes, and for a variety of purposes. Accordingly, usage of the medical device in a wide range of use cases is vital for understanding the (range of) normal operation values of the medical device. Often, a single instance of a medical device may only be used in one environment, in one mode and for one purpose. Thus, it may not be possible to determine the full range of normal operation values from usage data of one device. Accordingly, embodiments of the present invention overcome this issue by gathering usage data from many related medical devices in order to fully appreciate a wide range of operation conditions.

Additionally, the usage data of the medical device is compared to a standard operation policy, as well as the normal operation values. This may reflect the fact that a device is (or indeed, is not) intentionally configured to operate in a deviated fashion from normal/typical operation of the medical device. As such, a more accurate assessment of a deviation may be made, leading to a more appropriate occurrence of an operation issue signal.

When a discrepancy between the usage data and normal operation values is detected, an operation issue signal is generated. Such a signal may indicate to users, operators, and/or technicians that the medical device is operating in an erroneous way. Accordingly, this may indicate that there is a fault present, the nature of the fault, and a recommended course of action for correcting the fault (i.e. returning the medical device to a normal operation state). In more complex embodiments, the operation issue signal may be used directly to correct the fault (i.e. by changing operation parameters of the medical device).

Overall, the agent may be a piece of hardware installed on/near the medical device. Alternatively, the agent may be software-based, and installed within an operating system of the medical device. In any case, the agent may be appropriately provided in order to monitor the medical device (i.e. have access to system logs, sensors, etc.), and to also provide the operation issue signal.

In some embodiments, the agent further comprises a recommendation module configured to: process the usage data of the medical device and the usage data of the at least one other medical device in order to generate a best practice recommendation describing an operation mode for the medical device; and communicate the best practice recommendation to a user associated with the medical device.

Another benefit of obtaining information from other (related) medical devices, is that usage data of the devices may be assessed in order to determine which medical device is operating in an improved (i.e. more efficient, accurate, safe etc.) manner. For example, if it is discovered that certain operation parameters lead to a more accurate reading from the medical device, then it may be recommended to a user to use such operation parameters. Alternatively, if it is determined that operating the medical device at a lower temperature means that the medical device operates in a more efficient manner, then this may be recommended to the user.

Thus, the agent according to such embodiments provides a means for informing the user of how to operate the medical device in a more effective way.

In some embodiments, the network module may be configured to: establish a communication channel between the agent and at least one other agent, each other agent configured to obtain usage data of the at least one other medical device; and receive, via the communication channel, the usage data from the at least one other agent.

By configuring the network module in this way, the agent may be able to gather information from a large number of networked agents. As the agents may be networked networked, data may be automatically obtained from other agents, enabling the automatic generation of normal operation values.

Each agent may be similar to the agent of the invention (i.e. also gather usage data of other medical devices, compare usage data to determined normal operation values, and produce a operation issue signal) or may simply be a device capable of gathering usage data of another medical device.

In some embodiments, the analysis module may be further configured to: determine, based on the operation issue signal, a correction action describing a change to the medical device to reduce the discrepancy; and execute the correction action on the medical device.

After a discrepancy has been noted, a correction action may be determined to reduce such a discrepancy, potentially bringing the medical device back into normal operation. This may avoid the need for a skilled technician to manually inspect the medial device, and may obviate the need for correction by a skilled technician entirely.

In this case, the correction action may comprise at least one of a medical device parameter change, and a medical device operation order change.

Thus, the correction action may change the operation of the medical device in order to attempt to return the medical device back to normal operation.

Further embodiments provide that the analysis module may be configured to determine the correction action based on the operation issue signal and the usage data of the at least one other medical device.

One method of determining the correction action is based (at least in part) on the operation issue signal, which describes the discrepancy, and usage data of another medical device. Indeed, the other medical device may be within a normal operation value range. Thus, settings of the other medical device may be copied in an attempt to recue the discrepancy and reduce the medical device to a normal operation state.

In yet further embodiments, the analysis module may be configured to input the operation issue signal to a rectification machine learning module adapted to identify the correction action based on the operation issue signal.

Another method for determining the correction action may be by a machine learning algorithm. This may lead to a more complex and more effective correction action. Indeed, when multiple factors are contributing toward the deviation of the medical device from the normal operation values, it may not be clear how the operation parameters of the medical device should be changed. A machine learning algorithm may be able to determine solutions not immediately apparent.

In some embodiments, the analysis module may be further configured to: determine whether the correction action would alter manually set operational parameters of the medical device; and responsive to determining that the correction action would alter the manually set operation parameters, alerting a user associated with medical device of the correction action.

It may be the case that the medical device has a set of manually set operational parameters. This may be, for example, due to a special use case of the medical device, or an availability of certain resources (i.e. available power for the medical device, environmental factors, etc.). Thus, once the correction action is determined, it is beneficial to check whether there are any manually set parameters in order to avoid changes to the medical device not anticipated or wanted by the user. However, the user should also be alerted as to the impact of such manually set parameters so that they may make their own choice with regard to the preferred operation and normal operation trade-off.

In some embodiments, the interface may be configured to obtain the usage data from sensor logs of the medical device, and system parameter logs of the medical device.

Two specific sources of usage information may be sensor logs and system parameter logs. Thus, it may be beneficial as to the accuracy of the generated operation issue signal that the agent may access this information.

In some embodiments, the usage data may comprise at least one of: medical device usage patterns, medical device parameters, medical device behavior, and medical device best practices.

The usage data, in embodiments of the invention, may further comprise contextual data, including at least one of: physical parameters, and environmental parameters. In this case, the interface may be configured to obtain the contextual data from sensors of the medical device.

Indeed, a context of the medical device may impact the assessment as to whether the medical device is actually operating outside of a normal operation range. For example, if a room in which the device is situated is particularly hot, then this may account for a degradation in certain operational parameters of the device.

In some embodiments, the learning module may be configured to determine the normal operation of the medical device by inputting the usage data of the medical device and the usage data of the at least one other medical device to a reinforcement learning model adapted to identify a typical operation of a medical device based on operational parameters of a plurality of related medical devices.

In this way, a more accurate set of normal operation values may be determined.

In some embodiments, the analysis module is further configured to: generate, based on the operation issue signal, operation advice related to an operation mode of the medical device; and communicate the operation advice to a user associated with the medical device.

According to further examples in accordance with an aspect of the invention, there is provided a system for a monitoring a medical device, the system comprising: a first agent for monitoring a first medical device; a second agent for monitoring a second medical device, and wherein the network module of the first agent is configured to obtain usage data describing an operation of the second medical device from the second agent.

In this way, the invention may provide a network of agents capable of determining a normal operation value of the medical device that they are monitoring. This enables the automatic assessment of a number of different medical devices, reducing an amount of time spent by skilled technicians in diagnosing such devices. In some cases, this may also completely negate the need for a user to contact a technician to fix the device, and prevent unnecessary work for the technician.

According to additional examples in accordance with an aspect of the invention, there is provided method for monitoring a medical device, the method comprising: obtaining usage data describing an operation of a medical device; obtaining usage data describing an operation of at least one other medical device related to the medical device; determining normal operation values of the medical device based on the usage data of the medical device, and the usage data of the at least one other medical device; comparing the usage data of the medical device with the normal operation values of the medical device and a standard operation policy; and generating, based on the comparison, an operation issue signal describing a detected discrepancy between the usage data of the medical device with the normal operation of the medical device.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified representation of a network of local and global agents for monitoring medical devices;
Fig. 2 presents a simplified embodiment of detecting a deviation of a medical device from normal operation values using a reinforcement learning model;
Fig. 3 presents a simplified block diagram of an agent for monitoring a medical advice according to an embodiment of the invention;
Fig. 4 presents a simplified block diagram of a system for monitoring medical devices using agents according to another embodiment;
Fig. 5 presents a flow diagram of a method for monitoring a medical device according to a further embodiment; and
Fig. 6 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

The invention proposes concepts for monitoring medical devices. In particular, usage data of the medical device and other (related) medical devices is gathered. Tis data is used to learn normal operation values of the medical device. Accordingly, any deviations of the operation of the medical device from the normal operation values and a standard operation policy may be determined. The deviation is then used to generate an operation issue signal (i.e. a user alert, a correction action, etc.). In this way, operation issues may be automatically detected and potentially corrected at an early stage.

Embodiments of the invention provide a plurality of (smart) agents connected in a network for detection of wrong usage patterns of medical devices (i.e. malfunctioning of a medical device). Accordingly, more serious breakdowns of the medical devices/machines may be avoided, and in some cases the root problem for the incorrect usage pattern may be rectified. As a result, the invention may aid in the provision of an improved user (i.e. owner of the medical device) experience by reducing future issues with installed medical devices/machines, reducing the cost of replacement parts, and reducing an amount of service and maintenance overhead.

Put another way, embodiments of the invention detect incorrect usage pattern of a medical device, which enables mitigating actions to be taken in order to avoid future breakdowns and repair. Furthermore, service and maintenance costs may be reduced due to an automated nature of the invention, ultimately enabling an improved user/owner experience. On a larger scale, if agents of the present invention are provided to monitor medical devices, there may be a reduced workload for skilled technicians, so that more complex and/or serious issues may be focussed on. In the same way, the detection (and potential correction) of wrong/incorrect/abnormal usage patterns of the medical device (or individual components/parts therein) save cost, hassle and time of the user by eliminating future breakdowns, and the need for extensive part replacements.

According to particular embodiments, the benefits outlined above may be provided by a system including the following features:
(i) The capability to monitor usage of critical components/parts of the medical device by an agent implemented in hardware and/or software. The agent may continuously monitor and/or mine usage pattern data (i.e. from system logs and parameters), as well as/including physical and environmental changes (i.e. from sensors).
(ii) A connected network of agents for sharing usage data of medical devices, effectively providing a referential framework. The networked agents may be local (i.e. within one medical centre) or may be global (i.e. across many medical centres).
(iii) A learning module that may determine/learn a correct/typical/normal overall usage and performance policy of the medical device. In some cases, reinforcement learning among the agents in the same system to learn correct overall usage/performance policy may be employed to determine the normal operation values. Thus, any deviation of one of the agent's usage policy may impact the learning algorithm's convergence, resulting in instability that can trigger an operation issue signal/alert.
(iv) A means for rectifying a detected fault. For example, a configuration, usage pattern, behaviour of the medical device components may be altered. This alteration may be determined by reference to the usage data of other medical devices supplied by the networked agents.
(v) An adaptation capability such that the determined normal operation values may be adapted to new/different use cases of the medical device.

Embodiments of the proposed invention comprise software and/or hardware agents for monitoring medical devices that detect a deviation in a usage pattern of the medical devices, as well as abnormalities in parts/components of the medical device. Such deviations and abnormalities can lead to future breakdowns, and therefore early detection and appropriate action may avoid larger issues.

Indeed, one aspect of the invention is the provision of a set of smart software and/or hardware agents for monitoring medical devices. These agents may be deployed to monitor critical components of medical devices, and/or the medical device as a whole. The agents are configured to continuously investigate/monitor data of the medical device (i.e. usage data of the medical device/component thereof). The agents are configured to obtain usage data of other agents, so as to validate normal behaviour (i.e. check usage data of the device compared to normal operation values). This may be achieved by the establishment of a network, as explained in more detail below.

Put another way, each agent may be responsible for a component of the medical device. Each agents thus has a set of objectives related to usage pattern or behaviour of the component (i.e. a normal operation policy). This may include a knowledge base that helps in detecting the abnormal usage pattern of the medical device component. In some cases, the agent may be able to act to rectify detected issues prior to extensive damage.

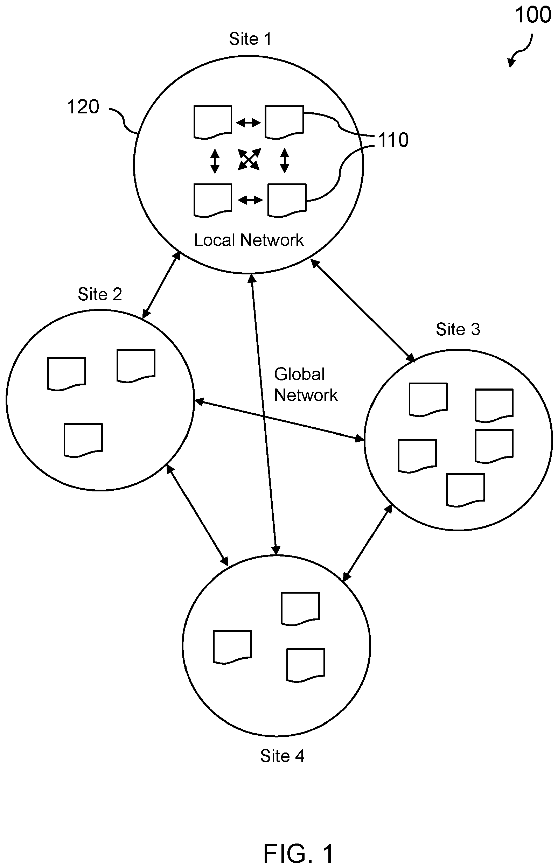

As presented in Fig. 1, the agents may be connected together to create a network 100. For example, agents 110 monitoring different medical devices in the same hospital may create a local network 120 (i.e. in site 1, 2, 3 and 4) and agents 110 from different hospitals may create a global network 100. Agents 110 can communicate to each other through the network connections, in order to share usage data of their respective monitored medical devices, and normal operation policies thereof. Indeed, localized configurations and best practices may be shared in the local network, while the global network provides generic configurations and best practices.

Each agent is configured to monitor the usage pattern/behaviour of the medical device/components, thus generating usage data of the medical device. The agents analyse the usage pattern data (from logs, system parameters), physical changes, environmental changes (can be from sensors) by applying data mining methodologies to extract important information for detection of an incorrect usage pattern.

The usage data mining process may involve the following steps:
(i) Data pre-processing: Data pre-processing involves noise removal, compensation for missing values, and structuring of the data.
(ii) Data transformation: This step involves transformation of the raw usage data into a more insightful data representation. Specifically, the data may be normalized, a dimension of the data reduced, and data features extracted.
(iii) Modelling data: The next step is to model the usage data and determine any pattern of the usage data. Different supervised and unsupervised techniques can be applied for this task, such as classification, clustering, regression etc.
(iv) Key insights/knowledge from the data: The key findings from the usage data that help in understanding the usage pattern of the medical device may then be provided.

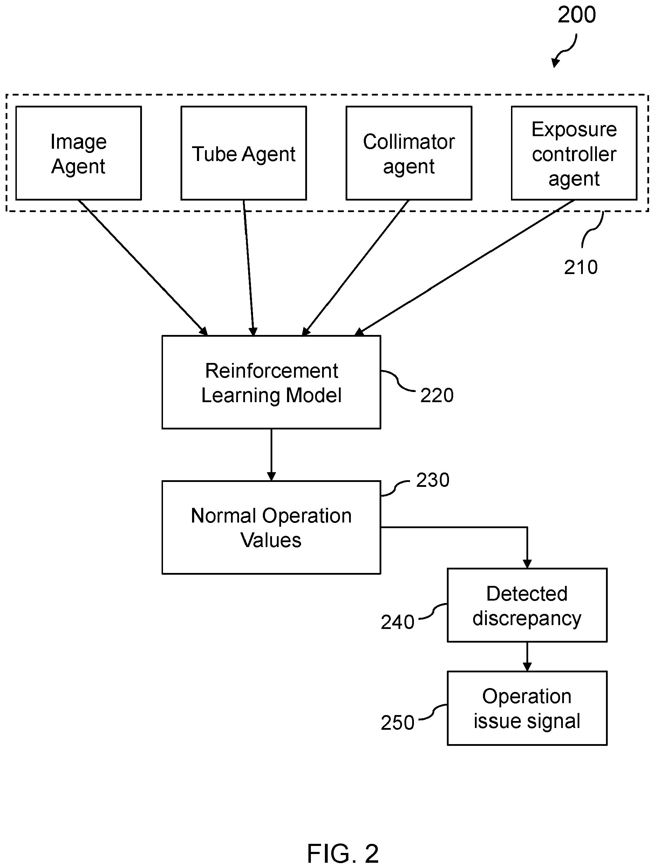

Once usage data of a plurality of medical devices is obtained, a wrong/incorrect usage pattern of the medical device may be detected. In specific embodiments, a reinforcement learning based methodology may be employed, as depicted in Fig. 2.

Specifically, a reinforcement learning model 220 is developed involving a plurality of agents 210 in the same medical device in order to ascertain (a range of) normal operation values 230 of the overall medical device. Any deviation of one of the agent's policy may impact the convergence of the reinforcement learning model 220, resulting in instability (i.e. a detected discrepancy). The example of Fig. 2 is of an x-ray machine, and shows agents 210 of different components of the x-ray machine inputting usage data to the reinforcement learning model 220. When one of the agents provides usage data that deviates from normal operation values 230, a discrepancy is detected 240, and an operation issue signal is generated 250.

In some embodiments, the agent may be authorized to perform certain rectification actions for an identified discrepancy/issue. In this case, usage and policy data obtained from other agents (via the network of agents) may be leveraged as a referential framework. The agent may rectify the fault based on the configuration of the medical device, a usage pattern of the medical device, and local configurations learned from agents where the medical device is running correctly.

In other words, embodiments of the proposed invention detect/identify usage patterns which deviate/diverge from the normal course of usage (i.e. normal operation values) suggested or practiced locally and/or globally. Based on this detection the agent may rectify such practices by changing certain parameters, a sequence of operations, a configuration of the medical device, etc.

However, it is important to provide a fail-safe technique in place before rectifying the medical device, and impose a control point on such decisions. There may be certain usage patterns/parameters set that are based on specific requirements of a user/institution. More particularly, a particular setup may deviate from a suggested/normal usage pattern. For example, there may be a certain electrical power limitation for the KV parameter of an x-ray/CT machine, and so the KV parameter may be limited to some pre-defined number (which deviates from the recommended guideline setting). This may result in reduced contrast images, but the user may be aware of, and accept, this limitation. In such a scenario, embodiments of the invention may not immediately rectify the detected issue by changing the parameter settings, but may instead simply suggest there is a need to rectify this usage pattern by changing the KV parameter to the user for their own decision making.

Accordingly, embodiments of the invention handle a correction action (i.e. a change to a usage pattern and medical device parameters) according to the following method:
(i) When an operation issue signal is generated, and a correction action determined, maintenance logs of the medical device are checked in order to clarify whether the correction action affects set parameters. It is also checked whether the set parameter serves an intentional purpose by the user.
(ii) If the correction action is likely to impact the set parameter, and the set parameter has a purpose, then the agent is prevented from performing the correction action. Instead, an alert may be generated, which provides information and a rectification recommendation to a user and/or technicians. The user and/or technician may then decide to accept or reject the correction action.
(iii) If the correction action is rejected, then the current usage pattern of the medical device may be flagged as a correct usage for that particular medical device (i.e. may be accepted as normal operation values). Thus, this incorrect usage may be effectively ignored.
(iv) Finally, any accepted correction action will be logged, so that the settings of the medical device may be rolled-back in future.

Moreover, the agent may be used to recommend best practices. Indeed, as some embodiments of the invention provide that agents from different sites are connected over a network (with continuous monitoring of respective medical devices), this information may be effectively utilised in order to identify the best practice/mode (i.e. most efficient, more effective, etc.) of using the medical device. Such a best practice recommendation may be provided to the user. Furthermore, the agents can also be used for education purposes. The user may be notified whenever a deviation from normal operation values is determined. The agents can communicate these situations to the user, including a type of deviation and an associated possible damage/impact that may be caused by the deviation.

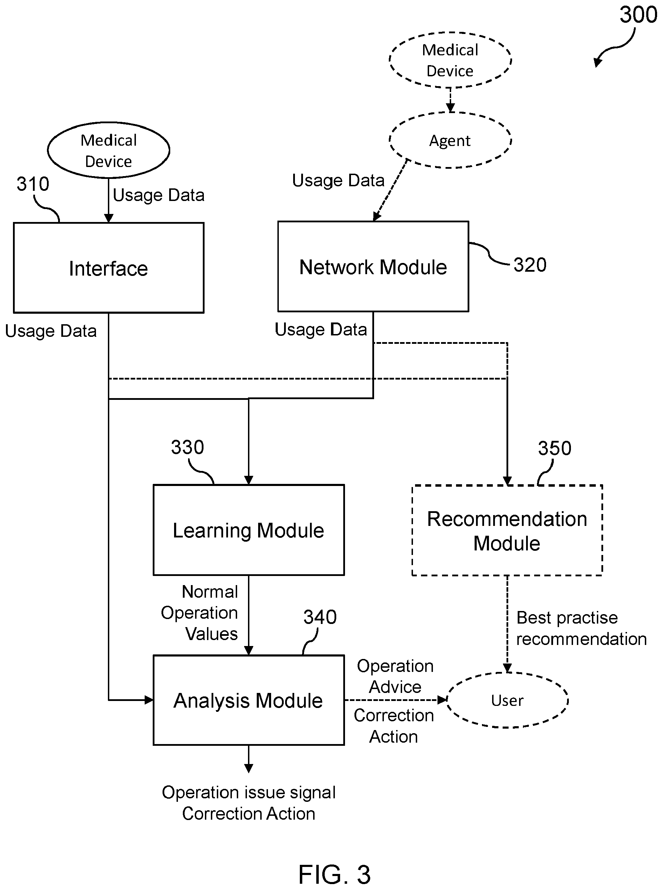

Turning now to Fig. 3, there is provided a simplified block diagram of an agent 300 for monitoring a medical device according to an embodiment of the invention. In particular, the agent 300 comprises an interface 310, a network module 320, a learning module 330 and an analysis module 340. In some embodiments, the agent 300 may further comprise a recommendation module 350. The agent 300 may be implemented in hardware and/or in software.

Overall, the agent 300 is configured to monitor a medical device, such as an x-ray machine, an MRI machine, an ECG monitor, or a component thereof. As such, the agent 300 gather usage data of the medical device, compares the usage data to a usual operation scheme, and outputs an operation issue signal based on any discrepancy/deviation from normal operation of the device. In this way, the agent 300 may alert users/technicians to present and potential problems/faults/issues with the medical device, provide advice regarding correction actions, and potentially rectify the problem in-situ.

Specifically, the interface 310 is configured to obtain usage data describing an operation of a medical device. The usage data includes information that indicates how the medical device is operating, for example including inputs, outputs and parameter values. Thus, a status/condition of the medical device may be determined from the usage data. The usage data may be obtained in real-time, or may be obtained in batches when the medical device state is to be determined.

The interface 310 may obtain or gather the usage data directly from the medical device. In some embodiments, the usage data may be retrieved directly from sensor logs of the medical device, and/or system parameter logs of the medical device. Alternatively, the interface 310 may obtain the usage data indirectly, for example from a repository of usage data information.

Further, the interface 310 may pre-process the usage data in order to eliminate noise, account for missing values, and extract useful information from the usage data.

In some embodiments, the usage data comprises at least one of: medical device usage patterns (i.e. times of use, length of use, frequency of use), medical device parameters (i.e. operating parameters), medical device behavior (i.e. reactions to inputs, effectiveness of outputs), and medical device best practices (i.e. a recommended operating scheme). It should be appreciated, however, that the usage data may include any information that describes how the device is operating, and that may indicate a state of the device.

In order to gain a greater appreciation of the operation of the medical device, the usage data may further comprise contextual data describing an environment/surroundings of the medical device. For example, the contextual data may comprise physical parameters (i.e. a placement of the device, a geography of the surroundings of the device) and/or environmental parameters (i.e. a room temperature, a humidity). In this case, the interface 310 may be further configured to obtain the contextual data from sensors of the medical device.

Moving on, the network module 320 is configured to obtain usage data describing an operation of at least one other medical device related to the medical device. The usage data may be of the same format of the usage data of the medical device, or may need to be adapted such that they may be compared. Accordingly, the usage data may be as described above.

The usage data of the related medical device(s) may be obtained directly from the other medical devices, or may be obtained from a repository of (historical) usage data of the medical devices.

Moreover, each of the at least one other medical devices are related to the medical device being monitored by the agent 300. That is, each other medical device(s) may be a of a same/similar model as the medical device (e.g. be a device of a same/similar design), of a same/similar type to the medical device (e.g. MRI machines), or may be related components in the same system as the medical device (e.g. an image controller and a collimator of an x-ray machine). Of course, the skilled person would appreciate the range of medical devices that may be considered related for the use of determining a normal operation of the medical device.

In some cases, the network module 320 is configured to establish a communication channel between the agent 300 and at least one other agent. In this way, the network module 320 may create a network of interconnected agents monitoring respective (related) medical devices. As explained in relation to Fig. 1, the agents may be local (i.e. within one site/medical centers), or may be global (i.e. across many sites/medical centers).

Each other agent in the network is configured to obtain/retrieve/gather usage data of the at least one other medical device. This may be achieved in a similar manner as the interface 310 of the present agent 300. In other embodiments, the agents may simply be sensors and/or system/parameter logs of other medical devices.

The communication channel may be implemented by any known wired and/or wireless communication schemes.

In the case that a communication channel is established. The network module 320 may receive, via the communication channel, the usage data from the at least one other agent. In some cases, a plurality of other agents may transmit usage data in real time to the network module 320. In other embodiments the network module 320 may receive usage data in batches when requesting usage data from the other agents. In yet further embodiments, the network module 320 may also transmit usage data of the medical device to the at least one other agents.

At least part of the usage data of both the medical device in question, and the other (related) medical devices, is then provided to the learning module 330. The learning module 330 is configured to determine (a range of) normal operation values of the medical device based on the usage data of the medical device, and the usage data of the at least one other medical device.

Indeed, it may expected that (given usage data of a large number of medical devices) an average of the usage data indicates what may be considered normal operation values. Put another way, a majority of medical devices may be expected to be operating within normal/typical/correct bounds. Such medical devices may define a range of operation values that may be considered normal.

In the case that the (related) medical devices are interrelated components in same/similar medical device systems, usage data of each of the components may be essential for understanding the interactions between the components, and thus typical overall medical device system behavior.

Moreover, the learning module 330 may be configured to determine the normal operation of the medical device by inputting the usage data of the medical device and the usage data of the at least one other medical device to a reinforcement learning model. The reinforcement learning model is adapted to identify a typical operation of a medical device based on operational parameters of a plurality of related medical devices. Indeed, the use of reinforcement learning was described in more detail in relation to Fig. 2.

Accordingly, reinforcement learning may be utilized in order to determine normal operation values, which may be used to identify atypical/incorrect behavior of the medical device. Alternatively, a machine learning model may be employed to determine normal operation values from usage data of related medical devices.

Furthermore, the agent 300 comprises the analysis module 340, which is configured to compare the usage data of the medical device with the normal operation values of the medical device (determined by the learning module 330) and a standard operation policy. That is, it may be determined whether the usage data falls within the (range of) normal operation values.

The usage data is also compared to a standard operation policy of the medical device, describing an expected typical operation of the medical device. In an embodiment, the standard operation policy is determined according to technical documentation. The standard operation policy may, for example, be obtained from the designer/manufacturer of the medical device.

In any case, this comparison enables the detection/identification of a presence (or lack of) of a discrepancy/deviation/irregularity between the normal operation values and the usage data of the medical device. An operation issue signal describing/indicative of the detected discrepancy may then be generated. The operation issue signal may be used to alert a user/technician, to determine potential rectification procedures, or to resolve the discrepancy itself.

In one instance, the operation issue signal may be used to determine a correction action. In this instance, the analysis module 340 is further configured to determine, based (at least in part) on the operation issue signal, a correction action describing a change to the medical device to reduce the discrepancy.

In other words, the correction action indicates a change to the medical device that may return the medical device to a normal/typical operation mode. By way of example, the correction action may comprise at least one of a medical device parameter change, and a medical device operation order change (i.e. a sequence change). Indeed, the correction action describes any change to the medical device that may result in a change in the behavior of the medical device.

The analysis module 340 may be configured to input the operation issue signal to a rectification machine learning module 330 adapted to identify the correction action based on the operation issue signal. Thus, machine learning may be leveraged in order to identify the most effective (or most likely to be effective) correction action for returning the medical device to normal/typical behavior.

In order to improve the identification of a correction action, the correction action may further be based on the usage data of the at least one other medical device. Indeed, a setup/configuration of another medical device falling within the scope of the normal operation values may indicate how the medical device may be altered (via the correction action) to return to normal operation.

The analysis module 340 may be further configured to execute the correction action on the medical device. Alternatively, the analysis module 340 may provide the correction action to another component that may actuate/implement the correction action, or may provide an indication of the correction action to a user/technician for implementation.

Alternatively, the correction action may not automatically be implemented/executed. Indeed, the analysis module 340 may further configured to determine whether the correction action would alter manually set operational parameters of the medical device. This may be achieved by comparing the correction action to system logs/history of the medical device.

Accordingly, responsive to determining that the correction action would alter the manually set operation parameters, the analysis module 340 may alert (i.e. transmit a notification) a user associated with the medical device (i.e. an owner, a technician, a current user) of the correction action. Thus, any accidental alteration of user preference parameters may be avoided, while still providing the user with the information required to make a judgement as to a trade-off between their preferences and the benefits of the correction action.

Furthermore, the operation issue signal may also be used to generate/identify operation advice. To do so, the analysis module 340 would be further configured to generate, based (at least in part) on the operation issue signal, operation advice related to an operation mode of the medical device. The operation advice may be related to a frequency, a length of use, a process order, system parameters, etc. Essentially the operation advice may provide the user/technician/owner with information that may keep the medical device operating within normal operation conditions/behavior, thus reducing the likelihood of potential damage. The operation advice is then communicated to the user/technician/owner of the medical device. Finally, the agent 300 may (optionally) further comprise a recommendation module 350 configured to process the usage data of the medical device and the usage data of the at least one other medical device in order to generate a best practice recommendation describing an operation mode for the medical device. Indeed, by taking into account all of the usage data from the (related) medical devices, it may be determined which operation scheme of the medical devices leads to most favorable medical device behavior (i.e. efficiency, effectiveness, lack of wear, etc.)

The best practice recommendation is then communicated to the user/technician/owner associated with the medical device, such that they may make their judgement as to whether to implement the best practice recommendation.

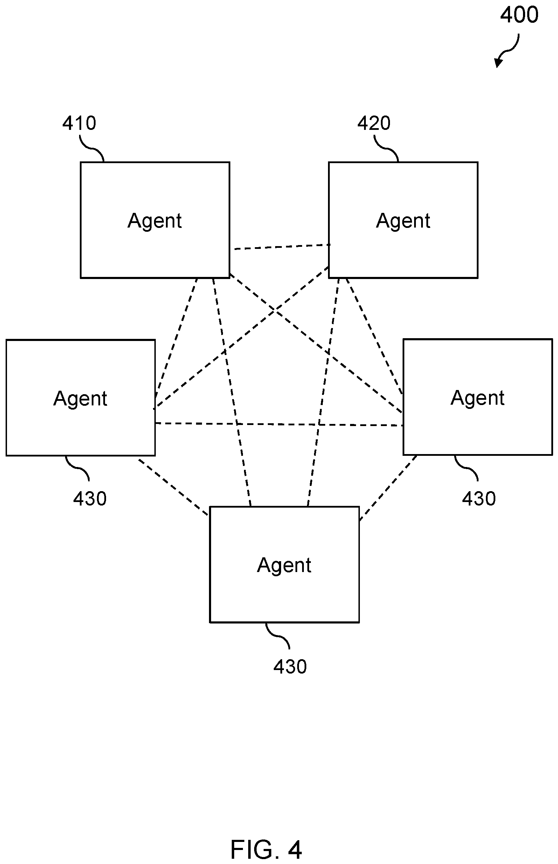

Fig. 4 presents a system 400 for monitoring one or more medical devices according to an exemplary embodiment.

In particular, there is provided a first agent 410 for monitoring a first medical device. The first agent 410 may be an agent 300 as described in relation to Fig. 3. There is also provided a second agent 420 for monitoring a second medical device, which may also be an agent 300 as described in relation to Fig. 3.

The first and second medical devices may be related devices. For example, the first and second medical device may be of a same or similar type, of a same or similar model, or be interrelated components of the same medical device system.

Furthermore, there may be provided additional agents 430 for monitoring additional (related) medical devices.

At least the network module of the first agent 410 is configured to obtain usage data describing an operation of the second medical device from the second agent 420. If additional agents 430 are provided, then the network module of the first agent 410 is configured to obtain usage data describing an operation of the additional medical devices from the additional agents 430.

Such usage data may be used to determine normal operation values, and so may be used to determine when a behavior of the first medical device is erroneous or atypical.

Also in some embodiments, the second agent 420 may be configured to obtain usage data describing an operation of the first medical device from the first agent 410. The second agent 420 may then use such usage data in a similar way to the first agent 410. This may also be true for the additional agents 430, if provided.

Essentially, the system of Fig. 4 provides the potential for a network for agents that share usage data, which is used to ascertain deviations of operational behavior of the medical devices from a normal operational range.

In some embodiments, there may be two different types of system. In a first instance, the agents may all be local agents forming a local network. In this case, the normal operational values will reflect, and preserve, a local usage pattern. The local usage pattern may be customized as per user requirements, and therefore preserving the pattern may be favorable. In a second instance, the agents may be global agents (i.e. across different sites, medical centers etc.) and so may form a global network. The global agent network may mean that the normal operational values reflect (and preserve) a generic usage pattern. The generic usage pattern may correspond to usage recommendations of the designer/manufacturer of the medical device.

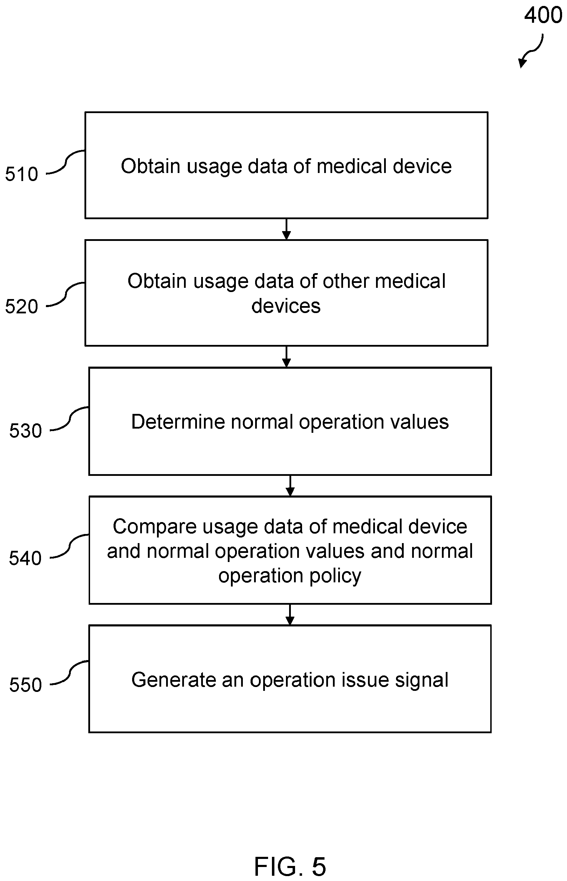

Fig. 5 provides a flow diagram of a method for monitoring a medical device according to an exemplary embodiment.

At step 510, usage data describing an operation of a medical device is obtained/acquired/gathered. This may be acquired directly from the medical device (i.e. from system logs) or from a repository of information. The usage data may e provided in real-time, or it may be obtained in bathes corresponding to (recent) history.

At step 520, usage data describing an operation of at least one other medical device related to the medical device is obtained. Similarly to step 520 this may be acquired from the other medical devices, or may be obtained from a repository. In some embodiments, the usage data of the other medical devices is acquired by establishing a communication network between agents monitoring the medical devices (i.e. forming a local and/or global network).

At step 530, normal operation values of the medical device are determined. The normal operation values are based (at least in part) on the usage data of the medical device, and the usage data of the at least one other medical device.

As such, a normal/typical/correct behavior of the medical device may be learnt based on the (range of) normal operation values. The normal operation values reflect a behavior of the medical device as it is operated in a state that may not lead to damage and/or faults.

At step 540, the usage data of the medical device is compared with the normal operation values of the medical device and a standard operation policy. Thus, it may be appreciated whether the medical device is operating in a normal state. In other words, a status of the medical device may be interpreted from the comparison between the usage data and the normal operation values.

Finally, at step 550, an operation issue signal is generated based on the comparison performed in step 540. The operation issue signal describes a detected discrepancy between the usage data of the medical device with the normal operation of the medical device.

Accordingly, the operation issue signal may be used to generate correction actions for reduction of the discrepancy, and operation advice to improve an operation of the medical device. Such actions and advice may be implemented automatically, or may require user/owner/technician input to implement.

As a result of the above described method, faulty behavior of the medical device may be automatically detected at an early stage. This may reduce the number and severity of faults, saving time and cost of both medical device owners and technicians.

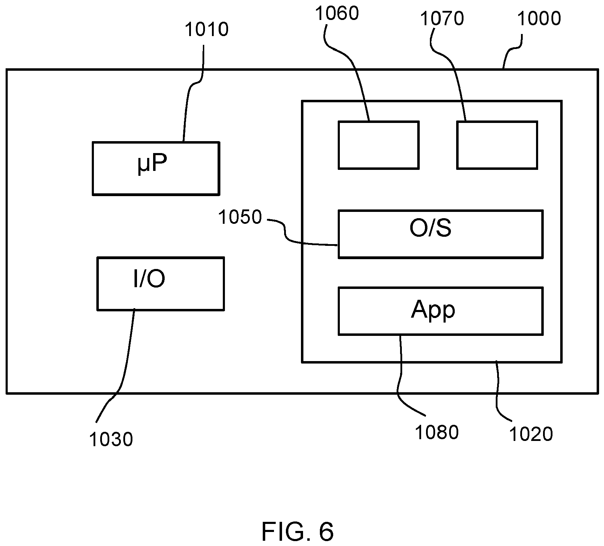

Fig. 6 illustrates an example of a computer 1000 within which one or more parts of an embodiment may be employed. Various operations discussed above may utilize the capabilities of the computer 1000. For example, one or more parts of a system for monitoring a medical device may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The method described in relation to Fig. 5, and the agents/systems described in relation to Fig. 3 and 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram Figs. 3 and 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. An agent (300) for monitoring a medical device, the agent comprising:
an interface (310) configured to obtain usage data describing an operation of a medical device;
a network module (320) configured to obtain usage data describing an operation of at least one other medical device related to the medical device;
a learning module (330) configured to determine normal operation values of the medical device based on the usage data of the medical device, and the usage data of the at least one other medical device, and
an analysis module (340) configured to:
compare the usage data of the medical device with the normal operation values of the medical device and a standard operation policy; and
generate, based on the comparison, an operation issue signal describing a detected discrepancy between the usage data of the medical device with the normal operation values of the medical device.

2. The agent of claim 1, further comprising a recommendation module (350) configured to:
process the usage data of the medical device and the usage data of the at least one other medical device in order to generate a best practice recommendation describing an operation mode for the medical device; and
communicate the best practice recommendation to a user associated with the medical device.

3. The agent of claim 1 or 2, wherein the network module (320) is configured to:
establish a communication channel between the agent and at least one other agent, each other agent configured to obtain usage data of the at least one other medical device; and
receive, via the communication channel, the usage data from the at least one other agent.

4. The agent of any of claims 1-3, wherein the analysis module (340) is further configured to:
determine, based on the operation issue signal, a correction action describing a change to the medical device to reduce the discrepancy; and
execute the correction action on the medical device.

5. The agent of claim 4, wherein the correction action comprises at least one of a medical device parameter change, and a medical device operation order change.

6. The agent of claim 4 or 5, wherein the analysis module (340) is configured to determine the correction action based on the operation issue signal and the usage data of the at least one other medical device.

7. The agent of any of claims 4-6, wherein the analysis module (340) is configured to input the operation issue signal to a rectification machine learning module adapted to identify the correction action based on the operation issue signal.

8. The agent of any of claims 4-7, wherein the analysis module (340) is further configured to:
determine whether the correction action would alter manually set operational parameters of the medical device; and
responsive to determining that the correction action would alter the manually set operation parameters, alerting a user associated with medical device of the correction action.

9. The agent of any of claims 1-8, wherein the interface (310) is configured to obtain the usage data from sensor logs of the medical device, and system parameter logs of the medical device.

10. The agent of any of claims 1-9, wherein the usage data comprises at least one of: medical device usage patterns, medical device parameters, medical device behavior, and medical device best practices.

11. The agent of claim 10, wherein the usage data further comprises contextual data, including at least one of: physical parameters, and environmental parameters, and wherein the interface is configured to obtain the contextual data from sensors of the medical device.

12. The agent of any of claims 1-11, wherein the learning module (330) is configured to determine the normal operation of the medical device by inputting the usage data of the medical device and the usage data of the at least one other medical device to a reinforcement learning model adapted to identify a typical operation of a medical device based on operational parameters of a plurality of related medical devices.

13. The agent of any of claims 1-12, wherein the analysis module (340) is further configured to:
generate, based on the operation issue signal, operation advice related to an operation mode of the medical device; and
communicate the operation advice to a user associated with the medical device.

14. A system (400) for monitoring a medical device, the system comprising:
a first agent (410) for monitoring a first medical device according to any of claims 1-13;
a second agent (420) for monitoring a second medical device according to any of claims 1-13, and
wherein the network module of the first agent is configured to obtain usage data describing an operation of the second medical device from the second agent.

15. A method (500) for monitoring a medical device, the method comprising:
obtaining (510) usage data describing an operation of a medical device;
obtaining (520) usage data describing an operation of at least one other medical device related to the medical device;
determining (530) normal operation values of the medical device based on the usage data of the medical device, and the usage data of the at least one other medical device;
comparing (540) the usage data of the medical device with the normal operation values of the medical device and a standard operation policy; and
generating (550), based on the comparison, an operation issue signal describing a detected discrepancy between the usage data of the medical device with the normal operation of the medical device.
